(19)

Europäisches Patentamt
European Patent Office
Office européen des brevets

(11) **EP 4 369 290 A1**

(12) **EUROPEAN PATENT APPLICATION**

(43) Date of publication:
**15.05.2024 Bulletin 2024/20**

(21) Application number: **22206737.3**

(22) Date of filing: **10.11.2022**

(51) International Patent Classification (IPC):
***G06T 7/00*** (2017.01)

(52) Cooperative Patent Classification (CPC):
**G06T 7/0016;** G06T 2207/10016;
G06T 2207/10116; G06T 2207/30101

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Siemens Healthineers AG
91301 Forchheim (DE)**

(72) Inventors:
• **NEUMANN, Dominik
91052 Erlangen (DE)**

• **TURCEA, Alexandru
105500 Busteni, Prahova (RO)**
• **ITU, Lucian Mihai
500294 Brasov (RO)**
• **PASSERINI, Tiziano
Plainsboro, 08536 (US)**
• **GULSUN, Mehmet Akif
Princeton, 08540 (US)**
• **BERGER, Martin
91088 Bubenreuth (DE)**

(74) Representative: **Kraus & Lederer PartGmbB
Thomas-Wimmer-Ring 15
80539 München (DE)**

(54) **DETERMINING ESTIMATES OF HEMODYNAMIC PROPERTIES BASED ON AN ANGIOGRAPHIC X-RAY EXAMINATION**

(57)     Various aspects of the disclosure generally pertain to determining estimates of hemodynamic properties based on angiographic x-ray examinations of a coronary system. Various aspects of the disclosure specifically pertain to determining such estimates based on single frame metrics operating on two-dimensional images. For example, the fractional flow reserve (FFR) can be computed.

FIG 6

EP 4 369 290 A1

**Description**

TECHNICAL FIELD

Various aspects of the disclosure generally pertain to determining estimates of hemodynamic properties based on angiographic x-ray examinations of a coronary system. Various aspects of the disclosure specifically pertain to determining such estimates based on single frame metrics operating on two-dimensional (2-D) images.

BACKGROUND

Morphological and functional assessment of coronary arteries from X-ray angiography examinations is used for the diagnosis of coronary artery disease (CAD), as it enables visual detection and quantification of coronary artery stenoses, wall abnormalities, or other hemodynamic properties. Furthermore, it is used to support treatment decisions, e.g., percutaneous coronary intervention (PCI) vs. coronary bypass grafting (CABG) and guide minimally invasive procedures (e.g., PCI) for CAD patients.

To this end, data obtained from angiography X-ray examinations is processed, such examinations comprising of multiple temporal sequences of 2-D images per patient acquired under various angulations/views.

SUMMARY

A need exists for advanced techniques of determining estimates of one or more hemodynamic properties based on 2-D images of an angiographic x-ray examination.

This need is met by the features of the independent claims. The features of the dependent claims define embodiments.

A computer-implemented method includes obtaining multiple 2-D images of an angiographic x-ray examination of a coronary system.

For example, such obtaining of the multiple 2-D images can include controlling an angiography system to acquire the multiple 2-D images. It would also be possible that the multiple 2-D images have been pre-acquired and are loaded from a memory or database or cloud storage.

The method further includes, for each one of the multiple 2-D images: in a respective processing pipeline, applying one or more analysis algorithms to the respective one of the multiple 2-D images to obtain a respective first-order estimate of the hemodynamic property of the coronary system.

The one or more analysis algorithms can determine estimates for one or more of the following: automatic view classification, vesselness/contrast detection, branch labeling, stenosis detection, stenosis grading, coronary tree tracing/reconstruction, artery lumen segmentation or fractional flow reserve (FFR) computation. The one or more analysis algorithms can be analytical algorithms or can be trained using machine learning techniques.

Some of the one or more analysis algorithms can determine an intermediate processing result that is required to then, at a second or later stage of the processing pipeline, determine the respective first-order estimate of the hemodynamic property. For instance, based on the automatic view classification as well as the vesselness/contrast detection, a stenosis may be detected and then subsequently graded. Then, the FFR computation can be executed based on such input. Thus, in other words, a hierarchy of processing can be implemented by multiple analysis algorithms along the processing pipeline, relying on the intermediate processing results.

Various hemodynamic properties can be determined using such techniques, e.g., the fractional flow reserve, detection of a stenosis, a grade of a stenosis, a count of stenosis, to give just a few examples. The hemodynamic property may include at least one of branch labels for coronary arteries of the coronary system, a segmentation between vessel and background, a topology of the coronary arteries of the coronary system, or a stenosis detection.

The particular type of hemodynamic property that is determined using the techniques disclosed herein is not germane to the functioning of the technology disclosed. This is because typically similar analysis algorithms to determine intermediate processing results are used for different types of hemodynamic properties. For instance, calculating an estimate of the FFR value would require an intermediate processing result that segments between vessel and background and captures the topology of the coronary arteries. Calculation of the estimate of the FFR value also requires the stenosis detection.

According to the disclosed examples, multiple (e.g., parallel) processing pipelines are established, one for each one of the multiple 2-D images. Each one of the multiple processing pipelines can include respective instances of the one or more analysis algorithms. This means that the same one or more analysis algorithms are executed for each 1 of the multiple processing pipelines, using different inputs. Thus, it is possible to calculate, based on a 2-D analysis of each one of the multiple 2-D images (separately), the first-order estimates of the hemodynamic property. Accordingly, multiple first-order estimates of the hemodynamic property are obtained, for each one of the multiple 2-D images. Each one of the one or more analysis algorithms can thus employ a single frame metric that operates based on individual ones of the multiple 2-D images and/or respective intermediate processing results derived therefrom.

The method further includes determining at least one second-order estimate of the hemodynamic property by consolidating the first-order estimate of the hemodynamic property obtained from each one of the multiple 2-D images.

Such techniques are based on the finding that the accuracy and/or reliability of the individual first-order estimates can be limited. This is because they are determined based on individual 2-D images, rather than on a cumulative/joint processing of the multitude of 2-D images. To mitigate such limited accuracy and/or limited reliability of the individual first-order estimates, the consolidation can take place. Various options are available for implementing such consolidation across the first-order estimates of the hemodynamic property. For instance, outliers may be removed. It would be possible to determine averages. It would be possible to consider a statistical distribution of the first-order estimates and then determine the second-order estimate based on such analysis. Details will be described hereinafter in this disclosure.

A processing device includes at least one processor and a memory. The at least one processor is configured to load program code from the memory and to execute the program code. Execution of the program code causes the at least one processor to obtain multiple 2-D images of an angiographic x-ray examination of a coronary system; and to apply one or more analysis algorithms to the respective one of the multiple 2-D images to obtain a respective first-order estimate of the hemodynamic property of the coronary system, for each one of the multiple 2-D images. Execution of the program code causes the at least one processor to further determine at least one second-order estimate of the hemodynamic property by consolidating the first-order estimate of the hemodynamic property obtained for each one of the multiple 2-D images.

A computer program or a computer-program product or a computer-readable storage medium includes program code that can be executed by at least one processor. Execution of the program code causes the at least one processor to obtain multiple 2-D images of an angiographic x-ray examination of a coronary system; and to apply one or more analysis algorithms to the respective one of the multiple 2-D images to obtain a respective first-order estimate of the hemodynamic property of the coronary system, for each one of the multiple 2-D images. Execution of the program code causes the at least one processor to further determine at least one second-order estimate of the hemodynamic property by consolidating the first-order estimate of the hemodynamic property obtained for each one of the multiple 2-D images.

It is to be understood that the features mentioned above and those yet to be explained below may be used not only in the respective combinations indicated, but also in other combinations or in isolation without departing from the scope of the invention.

BRIEF DESCRIPTION OF THE DRAWINGS

FIG. 1 schematically illustrates a 2-D image of an angiographic x-ray examination and segmentation of vessels of a coronary system according to examples.

FIG. 2 is a flowchart of a method according to various examples.

FIG. 3 schematically illustrates a processing device according to various examples.

FIG. 4 schematically illustrates a medical imaging system according to various examples.

FIG. 5 corresponds to FIG. 4 and illustrates a different setting of the medical imaging system according to various examples.

FIG. 6 schematically illustrates data processing including multiple processing pipelines according to various examples.

FIG. 7 schematically illustrates intermediate processing results of the processing pipelines according to various examples.

FIG. 8 schematically illustrates branch labels determined for multiple 2D images of an angiographic x-ray examination according to various examples.

FIG. 9 is a similarity metrics of the branch labels of FIG. 8.

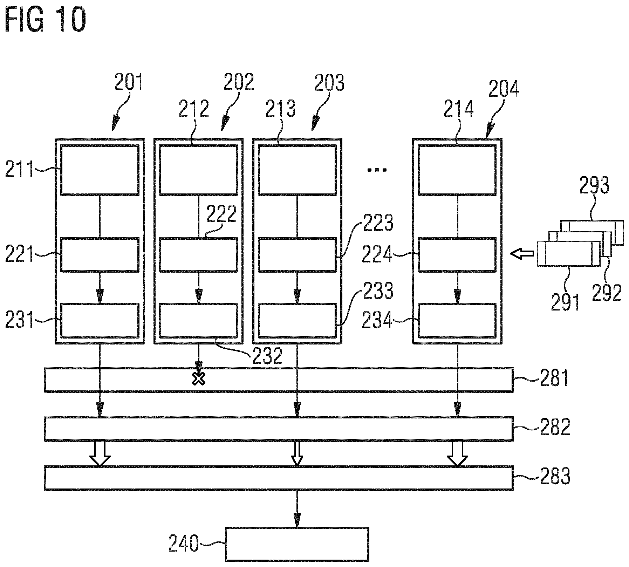

FIG. 10 illustrates data processing including multiple processing pipelines according to various examples.

DETAILED DESCRIPTION

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Various examples of the disclosure generally pertain to post-processing data obtained from an angiographic x-ray examination of a coronary system of a patient. Data obtained from the angiographic X-ray examination includes 2-D images. More specifically, multiple 2-D images are obtained. For example, the multiple 2-D images can be associated with multiple time points with respect to a contrast medium bolus. I.e., they can depict different states of the contrast agent flowing through the coronary system. Alternatively or additionally, the multiple 2-D images can be associated with multiple views of the coronary arteries of the coronary system that is investigated. In other words, it is possible that the multiple 2-D images include a temporal sequence of a given view/perspective. It is also possible that the multiple 2-D images include multiple temporal sequences associated with multiple views.

According to the examples disclosed here, the post processing is employed to determine the hemodynamic property, e.g., a FFR value with respect to a detected stenosis.

According to examples, multiple processing pipelines are set up that individually process each one of the multiple 2-D images. All processing pipelines can employ the same one or more analysis algorithms. Each processing pipeline can instantiate the one or more analysis algorithms and feed data based on the respective one of the multiple 2-D images to the respective instances of the one or more analysis algorithms. Each instance of a given analysis algorithm operates independently of other instances. Thus, 2-D confined processing is possible, using single frame metrics.

Such approach is computationally efficient, since 3-D dependencies are not natively considered. On the other hand, such approach can face difficulties regarding robustness and accuracy of the respective estimates of the hemodynamic property determined based on single-frame metrics.

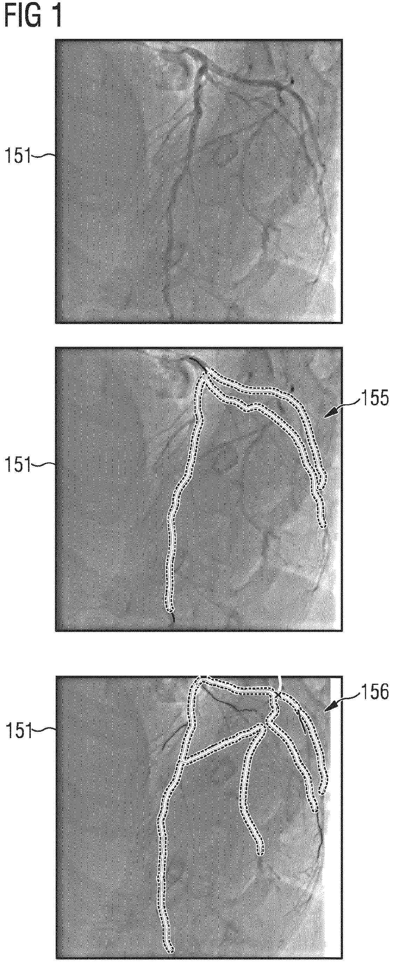

For illustration, FIG. 1 schematically illustrates a 2-D image 151 obtained from an angiographic x-ray examination of the coronary system of a patient. Illustrated in FIG. 1 are also to segmentations 155, 156 of the vessels of the coronary system. The segmentation 155 is correct while the segmentation 156 is incorrect.

Oftentimes, it is difficult, based on an isolated investigation of a single 2-D image, to detect whether an intermediate processing result of a corresponding processing pipeline that relies on one or more analysis algorithms and/or the final calculation result is reliable/robust or incorrect (cf. FIG. 1, incorrect segmentation 156). Accordingly, various techniques are based on the finding that by executing a consolidation of first-order estimates of the hemodynamic property that are obtained based on multiple processing pipelines that individually process each one of the multiple 2-D images it is possible to yield a second-order estimate of the hemodynamic property. This second-order estimate is more accurate and more robust than each individually first-order estimate.

Thus, in other words, according to examples, a two-stage processing is implemented. This is illustrated in connection with TAB. 1 below.

TAB. 1: Multi-stage processing to estimate a hemodynamic property.

| Stage | Explanation | Example details |
|---|---|---|
| Stage 1 | Determine multiple first-order estimates of hemodynamic property | In stage 1, each one of the multiple 2-D images is individually processed using one or more analysis algorithms. These one or more analysis algorithms form a processing pipeline. In other words, each one of the one or more analysis algorithms is instantiated multiple times, one for each one of the multiple 2-D images. This processing yields, for each one of the multiple 2-D images, a respective one of the first-order estimates of the hemodynamic property. |
| | | As a general rule, the type of analysis algorithms that are used to determine the first-order estimate can vary along with the variation in the calculation of the hemodynamic property. For instance, a framework for calculating a FFR value will be explained in further detail hereinafter with respect to FIG. 9; but other types of analysis algorithms may be used to estimate other hemodynamic properties. |
| | | On a general level, where multiple analysis algorithms are applied, it is possible that the stage 1 of the calculation employs one or more intermediate processing results. For instance, it would be possible that initially a segmentation between vessel and background is performed and then the vessels are mapped to predetermined tags/labels. Once the type of the vessel is known, it would be possible to attempt detecting a stenosis in each one of the appropriate vessels. This can include determination of one or more geometric properties such as a length of the respective vessel, a minimum diameter of the respective vessel in the respective 2-D image, a maximum diameter of the respective vessel in the respective 2-D image, and so forth. Thus, the intermediate processing results can be used as an input to a further analysis algorithm arranged downstream of the processing pipeline. |
| Stage 2 | Determine second-order estimate of hemodynamic property | Following stage 1 processing, stage 2 processing can then determine a second-order estimate (or multiple second-order estimates of the hemodynamic property by consolidating the first-order estimate of the hemodynamic property obtained for each one of the multiple 2-D images. |
| | | This enables to more accurately estimate the hemodynamic property. Outliers or wrong calculations based on individual 2-D images of the angiographic x-ray examination can be compensated or removed. |
| | | For instance, different types of consolidation can be employed to determine multiple second-order estimates. The multiple second-order estimates can thus give different approximations of the hemodynamic property, rooted in different techniques to implement a combined assessment of the first-order estimates. |

Hereinafter, various techniques of implementing a consolidation of the first-order estimates of the hemodynamic property to determine the second-order estimate, at processing stage 2 according to TAB. 1, will be disclosed. In other words, various techniques will be disclosed that enable to filter or aggregate the first-order estimates of the hemodynamic property. The goal is to select, compute, optimize or otherwise obtain one robust second-order estimate from the set of individual first-order estimates. A secondary goal of the proposed techniques is to ensure that the second-order estimate is deemed acceptable by the user following a visual inspection.

Such consolidation can be generally based on one or more comparative metrics that compare (i.e., put in relationship) one or more data structures of the processing pipelines associated with the multiple 2-D images (cf. TAB. 1: stage 1) with each other.

As a general rule, it would be possible that the one or more data structures that are considered as part of the comparative metrics for the consolidation include the first-order estimates of the hemodynamic property. Thus, the result values of the processing pipelines of stage 1, implemented for each one of the multiple 2-D images themselves, can be used. Alternatively or additionally to relying, for the consolidation, on the first-order estimates of the hemodynamic property, it would also be possible that the one or more data structures include at least one intermediate processing results of the one or more analysis algorithms and/or the input data, i.e., the multiple 2-D images.. I.e., an upstream processing variable of the respective processing pipelines can be used for the consolidation. In other words, it would be possible to focus on an analysis of the consistency of the intermediate processing results and/or data provided to the processing pipelines as input such as the 2-D images.

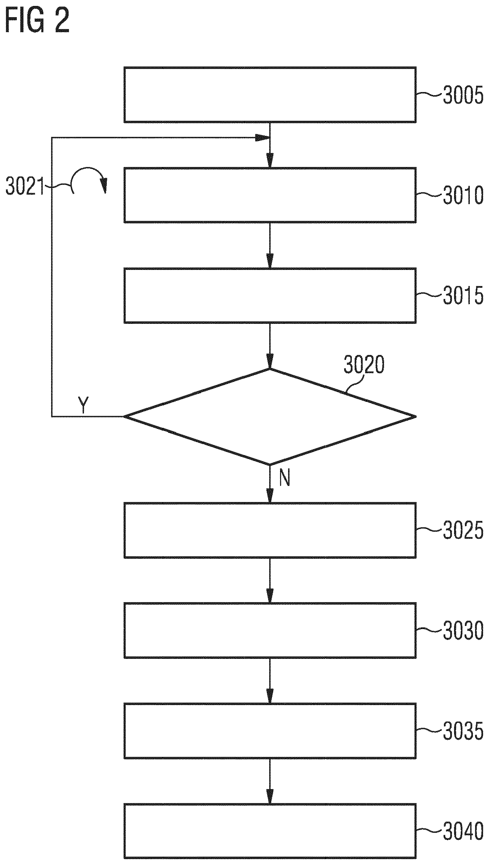

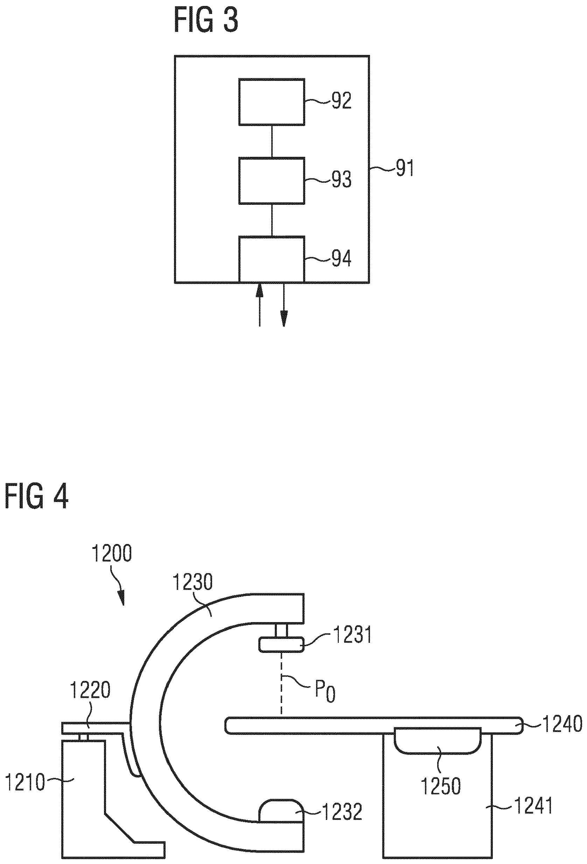

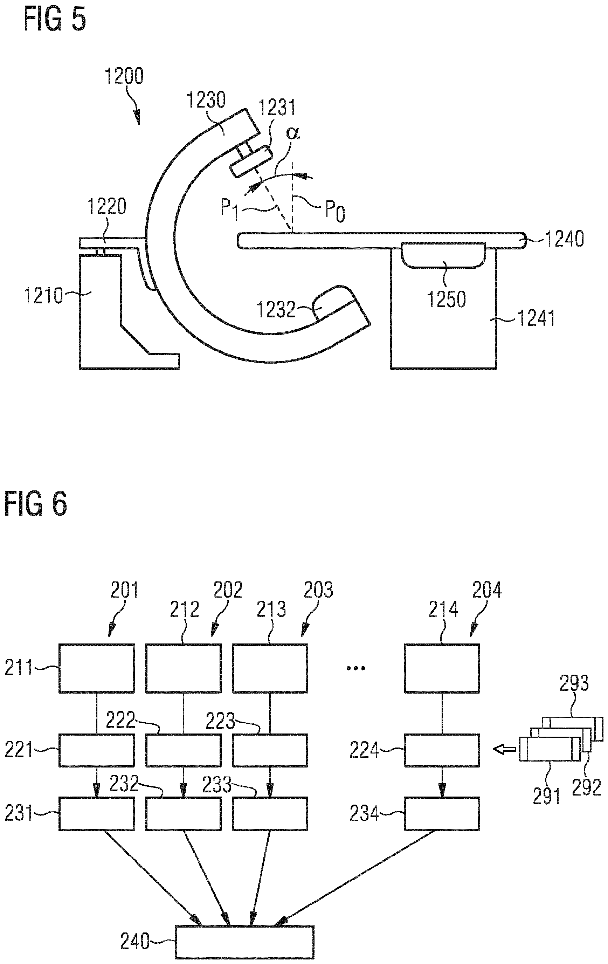

FIG. 2 is a flowchart of a method according to various examples. The method of FIG. 2 can be executed by a processor, upon loading program code from a memory and executing the program code. The processor could be part of an angiographic imaging system (cf. FIG. 4 in FIG. 5) or could be part of a computer.

The method of FIG. 2 generally pertains to post processing multiple 2-D images - e.g., associated with multiple time points with respect to a test bolus of a contrast medium and administered to a patient and/or multiple views - to obtain an estimate of the hemodynamic property.

Initially, at box 3005, multiple 2-D images of an angiographic x-ray examination of a coronary system are obtained, e.g., loaded from a hard drive/memory. It would also be possible that the angiographic imaging system is controlled to execute the angiographic x-ray examination, as part of box 3005.

Next, at box 3010, a current 2-D image for processing is selected from the set of obtained images of box 3005.

Then, at box 3015, in a respective processing pipeline associated with the currently selected 2-D image of box 3010, one or more analysis algorithms are used to obtain an associated first-order estimate of a hemodynamic property of the coronary system. A first one of the one or more analysis algorithms obtains the currently selected 2-D image as input. Where multiple analysis algorithms are employed, the processing pipeline includes calculation of one or more intermediate processing results. The respective processing pipeline can include a sequential use of multiple analysis algorithms, to consider intermediate processing results.

At box 3020, it is checked whether there is a further 2-D image which is to be processed. In the affirmative, a further iteration 3020 of boxes 3010 and 3015 is executed. Thus, the count of iterations 3021 corresponds to the count of 2-D images obtained at box 3005. Each iteration 3021 corresponds to a respective processing pipeline.

As will be appreciated, box 3015 thus operates an individual 2-D images. This corresponds to a scenario in which the one or more analysis algorithms are single frame metrics that operate based on individual ones of the multiple 2-D images.

Once all 2-D images have been processed, the method commences at box 3025. At box 3025, a second-order estimate on multiple second-order estimates of the hemodynamic property is determined.

This determination is based on the first-order estimates. It may be further based on another estimate of the hemodynamic property obtained from another angiographic examination, e.g., a prior examination using X-ray imaging or another imaging modality.

The determining includes consolidating the first-order estimates of the hemodynamic property obtained from each iteration 3021, i.e., obtained for each one of the multiple 2-D images.

For instance, where multiple second-order estimates are determined, this could be based on different comparative metrics that compare one or more data structures of the processing pipelines associated with the iterations 3021 with each other.

Where multiple second-order estimates are determined, it would be possible to rank the multiple second-order estimates with respect to each other at box 3030 and then, at box 3035, configure a user interface, e.g., a graphical user interface, to present the multiple second-order estimates in accordance with said ranking. For instance, such second-order estimates that have a higher ranking may be shown more prominently or may be shown at the top of the list, etc. Color may be used to indicate large or small ranking weights (e.g., green to red color scale), or the results could be sorted in a list according to their ranking weight. Thus, instead of one final result (i.e., a single second-order estimate), scenarios are possible where multiple final result candidates are determined (multiple second-order estimates), and in a final step, the user could manually pick one of them. The multiple results could be ranked by associated ranking weights.

The ranking can be based on the comparative metric. For instance, such comparative metrics that are known to operate well can be associated with a higher ranking of the thus obtained second-order estimate. For instance, the ranking could be based on a reliability value. For instance, it would be possible that the first order estimates are associated with respective reliability values, e.g., obtained as an output from the one or more analysis algorithms. Then, depending on the relative impact of each first order estimate on to the respective second order estimate, a larger or smaller reliability for the second order estimate may be obtained. Other ranking factors are, however, possible, e.g., in accordance with the user preference for certain comparative metrics employed to determine the respective second order estimates.

Alternatively or additionally to such ranking, the multiple second-order estimates could be presented clustered with respect to identified clusters. In other words, it would be possible to determine one or more clusters in a distribution of the multiple second-order estimates and then, at box 3035, configure the user interface to present the multiple second-order estimates in accordance with the one or more clusters. For example, such extension could be useful if the distribution of results is multi-modal (multiple clusters of similar results).

At box 3040, based on the one or more second-order estimates, it would be optionally possible to re-parameterize the one or more analysis algorithms that are used in the iterations 3021 based on the one or more second order estimates of the hemodynamic properties.

For instance, the determined weights for a weighted distribution or for a weighted combination of the first-order estimates, similarity scores, selections, etc. could be fed back to the one or more analysis algorithms (e.g., centerline tracing), which generated input data for the current module (e.g., FFR), in order to improve or correct the result of the preceding modules (e.g., correct the tracing result based). One or more iterations of the whole process (box 3005-3040) can be used: run module A -> determining criteria based on A -> use criteria to update results of preceding module B -> run module A with updated result of B -> ... This can help to improve the overall accuracy for the second-order estimate.

FIG. 3 schematically illustrates a processing device 91 according to various examples. The processing device 91 can be implemented by a computer or server. The processing device 91 includes a processor 93 and a memory 92. The memory 92 can store a program code. The processor 93 can load the program code. The processor 93 can execute the program code. Executing the program code causes the at least one processor 93 to perform techniques as disclosed herein, e.g., obtain multiple 2-D images, e.g., by retrieving such images via a communication interface 94 from an angiographic x-ray imaging system or by loading these images from a database or the memory 92; performing an analysis of the multiple 2-D images to determine an estimate of a hemodynamic property, e.g., a FFR value for a stenosis of the coronary system , branch labels for coronary arteries of the coronary system, a segmentation between vessel and background for vessels of the coronary system, a topology of coronary arteries of the coronary system, and/or a stenosis detection. ; implementing multiple processing pipelines for multiple 2-D images, e.g., using single frame metrics that operate based on individual one of the 2-D images.

FIG. 4 and FIG. 5 both show a medical imaging system 1200 (angiography imaging system). The medical imaging system 1200 is used to obtain multiple 2-D images. The medical imaging system 1200 includes a rotatable C arm 1230. X-ray emission means 1231 and X-ray detection means 1232 may be mounted on C arm 1230. In Fig. 4, C arm 1230 is in a neutral position $P_0$, i.e. X-ray emission means 1231 are located directly above a patient surface 1240. In Fig. 5, C arm 1230 and thereby X-ray emission means 1231 are rotated counter-clockwise with respect to neutral position $P_0$ of C-arm 1230 in Fig. 5 to a position $P_1$. The angle between position $P_0$ and position $P_1$, as indicated in Fig. 5, is referred to as the angiography angle or the fluoroscopy angle, depending on the medical imaging process. This angle defines the view of 2-D images that are acquired in the corresponding setting. It will be understood that the neutral position may be used as an imaging position. In such a case, the angiography angle is 0°. Further, in case of a single axis angiography system, the neutral position is typically defined as shown in Fig.4. In multiple axis angiography systems, additional C arms may be present, such as a ceiling mounted C arm. In such a case, the neutral position may be defined as the position in which X-ray emission means 1231 and X-ray detection means 1232 are at the same level as a patient, on patient surface 1240.

While the above definition of the angiography angle is based on the position of X-ray emission means 1231, the angiography angle may analogously be defined based on the position of X-ray detection means 1232.

For example, angulation planning (i.e., selection of the appropriate angiography angle) may be performed based on the computed tomography angiography (CTA) data. This can help to select the appropriate views for which 2-D images are acquired by the angiography measurement; thereby helping to facilitate robust consolidation of first-order estimates obtained for the multiple 2-D images having multiples views.

A contrast medium dosage may indicate the dosage of radio-opaque contrast medium administered to a patient in order to render the vessels of the patient visible during the imaging method. The contrast medium dosage may be measured in milliliters per kilogram of body weight. In the case of contrast media including iodine, the contrast medium dosage may also be measured in milligram iodine per kilogram of body weight. At some point, a bolus of the contrast medium is injected into the blood vessel system of the patient. A time offset with respect to this injection defines a timing of respective 2-D images.

FIG. 6 schematically illustrates aspects with respect to multiple processing pipelines 201-205. Each one of the processing pipelines 201-204 corresponds to an iteration 3021 of FIG. 2. Each processing pipeline 201-204 is associated with a respective 2-D image 211-214 that is then processed using one or more analysis algorithms 291-293. As part of this processing, one or more intermediate processing results 221-224 are obtained. Next, multiple first-order estimates 231-234 of the hemodynamic property, e.g., fractional flow reserve value, are obtained (cf. box 3015). The multiple first order estimates 231-234 are then consolidated to determine a second order estimate 240 of the hemodynamic property (cf. FIG. 2, box 3025).

Next, multiple options for consolidating multiple first order estimates of the hemodynamic property to determine the second order estimate of the hemodynamic property (cf. FIG. 2: box 3025) will be explained.

For instance, in a one implementation, the consolidating is based on a combination or weighted combination - e.g., an average or mean - of the first-order estimates of the hemodynamic property.

If weights are used, the weights of the weighted combination can be set based on one or more comparative metrics. For instance, weights could be set based on one or more similarity measures between intermediate processing results, cf. TAB. 2 below.

As a general rule, such weights can strengthen or weaken the impact of the respective first-order estimate on the at least one second-order estimate of the hemodynamic property ("soft filter"). For instance, weights can take different continues values, depending on the respective impact. It would also be possible to completely suppress any impact of the respective first-order estimate of the hemodynamic property on the at least one second-order estimate of the hemodynamic property ("hard filter").

Next, a further example of implementing a comparative metrics will be explained. Without loss of generality, assume that the result $r^i$ of a processing pipeline (i.e., the first-order estimates 231-234 of the hemodynamic property) on a 2-D image $f^i$ 211-214 is a scalar. Then, to consolidate a set of results $R=\{r^1, ..., r^n\}$ resulting from input data for multiple individual 2-D images $F=\{f^1, ... f^n\}$ into a single robust result $r^*$ (i.e., the second-order estimate 240), a comparative metric that includes a statistical analysis can be performed on the first-order estimates, assuming a reasonable sample size n (number of 2-D images).

One option is to fit a statistical model (e.g., normal distribution, Gaussian mixture model, ...) to R (i.e., the distribution of the first-order estimates 231-234) and select or compute the most probable value as the second-order estimate 240 according to the properties of that distribution . In the case where a normal distribution can be assumed, the mean value is a good candidate for the second-order estimate 240. In the presence of noise, bias or outliers, alternatives such as the median value could be more meaningful. I.e., an outlier detection based on the statistical analysis could be used. In addition, explicit outlier detection and removal could be performed to remove potentially erroneous results prior to aggregation. As an example, the "random sample consensus" algorithm (RANSAC) can be used to identify outliers.

However, there is no guarantee that assumptions of the employed outlier detection method are valid (e.g., a small minority of alleged "outliers" could in fact be the more accurate results, and/or a majority of consistent results could in fact all be wrong). Alternatively, the minimum, maximum or percentile-based results could be used and yield a more meaningful second-order estimate 240 under specific circumstances. The variability of the results (e.g., standard deviation) could be an indication for the confidence, which could be passed on to the user.

Above, scenarios have been disclosed in which the one or more comparative metrics include a statistical analysis of a distribution of the first order estimates. It is possible, that the distribution is weighted. I.e., weights can be associated with each first order estimate and then the distribution can be calculated by considering such weights. For instance, a statistical model can be fitted to the first order estimates taking into consideration tolerances that are associated with the weights. The weights, in turn, can be determined based on one or more further comparative metrics, e.g., based on an intermediate processing result of the processing pipelines or the 2-D images, e.g., a quality of the images that are used for the calculation. For example, techniques to calculate the weights that have been disclosed above in connection with the weighted combination of the first order estimates can also be employed to weigh the statistical distribution.

Alternatively or additionally to such statistical analysis of the first-order estimates 231-234, it would also be possible to consider similarity measures between pairs of these first-order estimates 231-234. For instance, it could be determined where the certain first-order estimates significantly deviate from the first-order estimates and then determine whether such deviation is indicative of an outlier. This could also be based on similarity measures between pairs of corresponding intermediate processing results. Inconsistent input or inconsistent intermediate results a processing pipeline. I.e., the respective first-order estimate can be corrupted due to bad input or intermediate processing results.

Similarity measures for certain input data/2-D images or intermediate processing results can help recognize potential outliers. Similarity between pairs of 2-D images and/or pairs of intermediate processing results can be considered.

As a general rule, the intermediate processing results can be selected from a group including: a length of a main branch of vessels the coronary system; an average diameter of a selected one of a vessel of the coronary system; a maximum diameter of the main branch of the coronary system; a maximum diameter of a stenosis at the main branch of the coronary system; an integral computed along the main branch of the coronary system; a number of detected stenosis; a position of a proximal clipping point; a position of a distal clipping point; a position of a catheter tip; a position of centerline trace of the main branch of the coronary system; a number of bifurcations of the coronary system; positions of the bifurcations; a number of branches of the coronary system; a position of the branches of the coronary system; a number of detected stenoses; a position of the detected stenosis; a consistency between heat maps.

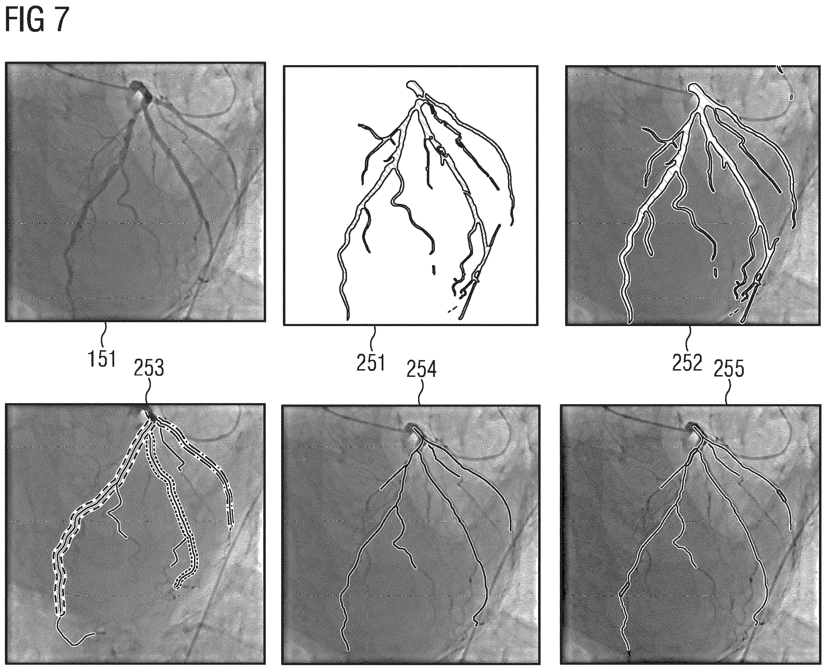

For further illustration, certain intermediate processing results that may be considered in such similarity measure are shown in FIG. 7.

FIG. 7 illustrates example intermediate processing results 251-255. Illustrated is an example 2-D image 151. Branch labels 251 can be calculated as an example intermediate processing result, identifying different coronary arteries. A vesselness 252 is another example of an intermediate processing result, e.g., including segmentation of background and foreground. Branch selection 253 can determine an example intermediate processing result, i.e., defining different branches of coronaries of the coronary system. A coronary tree/topology 254 is another example of an intermediate processing result. Finally, stenosis detection 255 can be determined as an intermediate processing result.

Specifically, FIG. 7 illustrates an example for determining estimates of FFR. For example, as highlighted in FIG. 7, as an intermediate step during the computations of each processing pipeline, certain branches are selected (e.g., main and side branches, which are fed to the FFR solver). Here, a similarity score between pairs of branch selection results from two processing pipelines can be exploited to identify inconsistencies and outlier, as similarity measure of a comparative metric. Comparable similarity scores can be established for other input data and/or intermediate processing results (as shown above). A combination of multiple similarity scores can help to increase accuracy of outlier detection, etc. Similarity scores can be used to influence the consolidation. For example, a statistical analysis may be used (as explained above) where the distribution is weighted based on such a comparative metric that employs similarity measures. For example, the first-order estimate associated with a given 2-D image that has an intermediate processing result which has a high similarity score to most other 2-D images receives a larger weight. For example, soft and hard filter criteria can be defined based on the individual similarity metrics.

Next, some concrete examples of intermediate processing results that can be considered as part of a comparative metric that considers a similarity measure are summarized in connection with TAB. 2.

TAB. 2: Examples of intermediate processing results of the processing pipelines that can be used by one or more comparative metrics for evaluating respective similarity measures. In certain variants, it is possible to calculate multiple similarity measures based on different examples as listed here and/or further examples. Sometimes, similarity measures may be selected specifically for certain 2-D images that are associated with a given view of the coronary system. Some similarity measures may be used for a first view and may not be used for a second view. For instance, a direct application of a location-based similarity measure may be invalid due to potentially large discrepancy in the appearance of the coronary tree, perspective for shortening, overlaps, in-between pairs of 2-D images associated with different views.

| | Intermediate processing result serving as a basis of the similarity measure | Example details |
|---|---|---|
| 1 | Length of main branch | The length of the main branch determined - as an intermediate processing result by a respective instance of the processing pipeline - for a first one of the multiple 2-D images is compared with the length of the main branch determined as the intermediate processing result using a respective processing pipeline for a second one of the multiple 2-D images. In such context, as a general rule, it would be possible that the length of the main branch (similarly, the length of any vessel of the coronary system) is compensated with respect to a perspective foreshortening that is associated with the respective view of the respective 2-D image. For instance, where the first and second images for which the length of the vessel, specifically the length of the main branch, is compared are associated with different views of the coronary system (cf. FIG. 4 in FIG. 5), this can result in different perspective foreshortenings associated with those views. To enable the comparison of the accurate length measures, this perspective foreshortening can be compensated for. This is possible under knowledge of the angle alpha. |
| 2 | Average diameter of vessel | For instance, the average diameter of along the first 1 cm (or another predetermine length) could be considered. This is based on the following finding: due to the proximal overlap, especially on the left coronary artery, the proximal segmentation may vary considerably, even though the proximal clipping point (i.e., start of segmentation) is consistent |
| 3 | Minimum diameter on main branch | The main branch can be identified and then along the entire length of the main branch, the minimum diameter of the main branch can be extracted. |
| 4 | Maximum %diameter stenosis on main branch: | Here, stenoses detected by the solver pre-processor are used: %DS=(1-d_min/d_healthy)*100, where d_min is the minimum diameter of the stenosis, and d_healthy is the healthy diameter, computed as average of the diameter at the start and the end of the stenosis. %DS is computed for all stenoses on the main branch, and the maximum %DS is considered. |
| 5 | integral computed along the main branch | This serves as a surrogate for viscous pressure loss along the main branch |

(continued)

| | Intermediate processing result serving as a basis of the similarity measure | Example details |
|---|---|---|
| 6 | Number of detected stenosis | For instance, the total count, count per branch or per branch segment may be considered. |
| 7 | Proximal clipping point | Similarity / consistency of a position of proximal clipping points (start of segmentation) be determined |
| 8 | distal clipping point | Similarity / consistency of a position of distal clipping points (end of segmentation/reported FFR location) can be determined |
| 9 | Catheter tip location | The similarity / consistency of a position of the catheter tip location can be considered |
| 10 | Main branch centerline trace | The shape of the main branch centerline trace can be compared. |
| 11 | Branch labeling | The consistency between branch labels (and optionally associated positions) in a given vessel, e.g., the main branch of the coronary system, can be considered.<br><br>An example implementation of determining the position of branches having a certain label is illustrated in connection with FIG. 8. FIG. 8 illustrates the branch label maps 361-365 associated with five different 2-D images, e.g., having different views. Illustrated are the branch labels as obtained from a respective analysis algorithm for a main branch 351, a first side branch 352, and a third side branch 353. The results are comparable and similar to each other for the branch label maps 361, 362, 363, as well as 365; however, the branch label map 364 has an interchanged branch labeling for the main branch 351 in the first side branch 352. This yields a similarity matrix including similarity measures between pairs of the branch label maps 361-365 as illustrated in FIG. 9. For instance, a similarity matrix according to FIG. 9 could help to identify the intermediate processing results 364 as faulty; thereby reducing (soft filter) or even excluding/suppressing (hard filter) the impact of the corresponding first-order estimate of the hemodynamic property onto the second-order estimate of the hemodynamic property. |
| 12 | number/locations of bifurcations in extracted coronary tree | The number of bifurcations, i.e., splitting of a single vessel into two or more vessels, can be counted. A position/location of these bifurcations can be considered in a respective 2D map. |
| 13 | Consistency of number/ locations of segments in extracted coronary tree | |
| 14 | Consistency of number/ locations of detected stenoses | |
| 15 | Heat map | Example heatmaps include vesselness, labeling, etc . A example metric to determine a similarity between heatmaps would be DICE score. |

As will be appreciated from TAB. 2, some of the intermediate processing results are associated with a position of respective features. Examples would include the position of the proximal clipping point, the position of the distal clipping point, the catheter tip location, or a comparison of spatial heat map. As a general rule, if the at least one similarity measure includes a position-based similarity measure, the position-based similarity measure can be determined in accordance with deformation fields between the views associated with respective pairs of the images. Thus, offsets in positions or spatial coordinates that are due to different views is of the underlying pair of 2-D images can be compensated for. In other words, for those similarity measures that are based on intermediate processing results that are related to spatial location of branch/points/... in the image coordinate system, it is possible to incorporate known or estimated deformation fields between the views that are being compared. This can be applicable even where nominally the same view is used for the compared 2-D images. In particular, this may help mitigate effects of motion between respective timestamps of the 2-D images on the metrics. Motion is inherent in temporal angiography sequences due to heart contraction, breathing, table motion, etc. In particular, let $T^{i,j}$ denote the transformation/mapping (e.g., deformation field computed by image-to-image registration method) that maps frame $f^i$ to frame $f^j$. Then prior to computing similarity to a location-based criteria such as the location of the catheter tip, denoted $c^i$ and $c^j$ for the two frames, one could first apply the transformation to $c^i$ to yield $c^{i*} = T^{i,j}(c^i)$, then compute similarity between $c^{i*}$ and $c^j$. This would reduce impact of motion on the computed similarity.

Above, scenarios have been disclosed that rely on the calculation of one or more similarity measures between pairs of intermediate processing results, for the consolidation of the first-order estimates of the hemodynamic property. Next, various further variants for implementing the consolidation will be disclosed.

Alternatively or additionally, in another variant of the implementation of the consolidation, analysis algorithms such as centerline tracing, lumen segmentation and stenosis detection can produce uncertainty estimates (uncertainty level) which can be then incorporated as soft criteria (e.g., weights for a distribution or for a weighted combination) in the consolidation or as hard criteria (suppressing any impact of the respective first-order estimate) if the uncertainty is above a certain level. For instance, machine learning algorithms can be used that output, in addition to the first-order estimate, also an uncertainty level. Also, analytical algorithms are known that are able to calculate the uncertainty level associated with such first-order estimates of the hemodynamic property. Then, it would be possible that, e.g., a first-order estimate that is associated with the comparatively large uncertainty level is weighted so that its impact onto the at least one second-order estimate is reduced if compared to an impact of another first-order estimate that this is associated with a small uncertainty level.

Alternatively or additionally, in another variant of the implementation of the consolidation, out-of-distribution likelihood scores may be used. Out-of-distribution likelihood score determine whether the input data for a certain analysis algorithm, for a certain 2-D image, is out-of-distribution. The likelihood score can be then incorporated as soft criteria (e.g., weights for a distribution or for a weighted combination) in the consolidation or as hard criteria (suppressing any impact of the respective first-order estimate) if the likelihood is above a certain level.

Alternatively or additionally, in another variant of the implementation of the consolidation, it is possible to take into account one or more properties of the input to the processing pipelines, specifically one or more properties of the multiple 2-D images. For instance, the consolidating can be based on an image quality score determined for each 1 of the multiple images. Various types of image quality scores are conceivable. For instance, it would be possible to determine the presence or absence of artifacts, e.g., known distortions of the images such as blur or noise.

Alternatively or additionally, in another variant of the implementation of the consolidation, the consolidating is based on the user input obtained from a human-machine interface. For example, it would be possible that a user may inspect the multiple 2-D images and then select certain images to be excluded from the calculation of the second-order estimate of the hemodynamic property. Then, a respective weight may be used that suppresses any impact of the respective first-order estimate (if even calculated) of the hemodynamic property on the at least one second-order estimate of the hemodynamic property. It would also be possible that the user can inspect one or more intermediate processing results (e.g., one or more intermediate processing results as presented in connection with FIG. 7) and based on such inspection increases or decreases (soft filter) or even suppresses (hard filter) the impact of one or more first-order estimates of the hemodynamic property onto the second-order estimate of the hemodynamic property.

The consolidation relying on user input is based on the finding that 2-D images having certain views are challenging to process fully automatically due to aspects like vessel overlap, stenosis not being visible, etc. As a result of the robustness analysis, certain user inputs may be desired. For example, if the distal end of the segmentations varies significantly between frames, and no clear outlier detection can be performed, the user may be asked to provide input in terms of clicking a desired distal end location on one frame which can then be propagated on other frames. Hence, minimal input provided by the user may significantly increase the robustness and the accuracy of the final results.

Alternatively or additionally, in another variant of the implementation of the consolidation, when there is a preoperative computed tomography angiography (CTA) measurement data available, a weight for the respective first-order estimate impact onto the second-order estimate impact can be determined based on the co-registered angiography and CTA image segmentation/findings. For example, segmentation results from multiple angio 2-D images in the cardiac cycle can be co-registered to CTA coronary segmentation in order to detect frames with outlier segmentation results in angio. The same applies for intravascular images (OCT / IVUS).

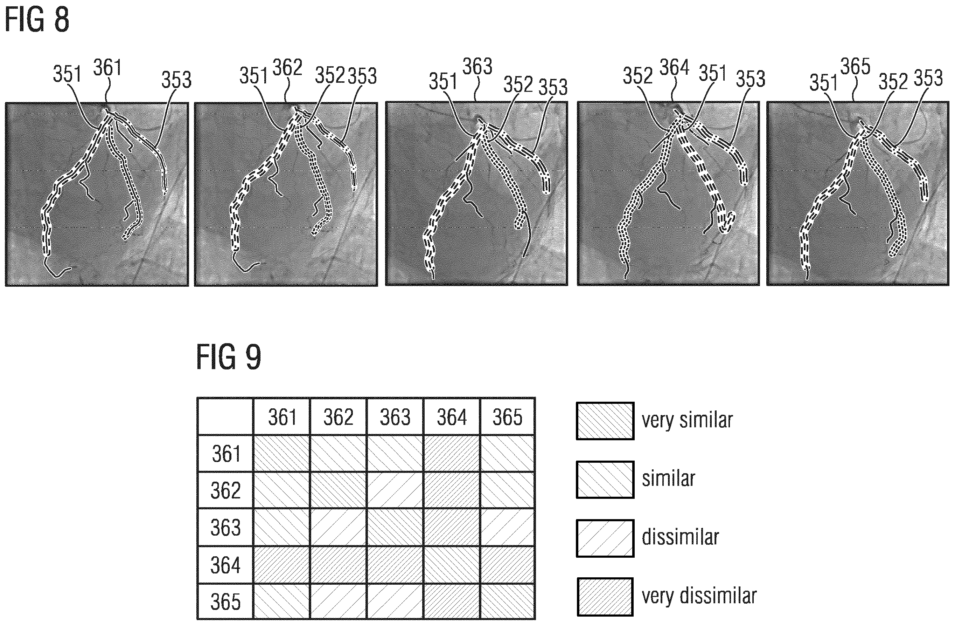

Above, various examples with respect to determining multiple first-order estimates of the hemodynamic property and consolidating the multiple first-order estimates determined the second order estimate of the hemodynamic property have been disclosed. Next, a specific example for calculating the FFR value as hemodynamic property will be discussed in connection with FIG. 9. FIG. 9 also illustrates a specific implementation of the consolidation of the multiple first-order estimates 231-234.

FIG. 9 generally corresponds to FIG. 1. Each processing pipeline 201-204 determines a respective first-order estimate 231-234 of the FFR. This is based on respective 2-D images 211-214. Each processing pipeline 201-204 can rely on multiple intermediate results 221-224 (FIG. 9, for sake of simplicity, only a single intermediate processing results 221-224 per processing pipeline 201-204 is shown).

Each processing pipeline 201-204 can extract a respective 2-D anatomical model. The patient specific arterial geometry is segmented on a 2-D angiographic image. The lumen border and the centerline are then used to define a two-dimensional anatomical model of the arteries of interest. The 2-D anatomical model is represented as a network of branching centerlines. A piecewise linear approximation to the centerline is considered, with a constant spacing of 0.5mm. A point-wise estimation of the vessel radius is available for the entire vascular tree, as a by-product of the centerline computation. Optionally, the centerline discretization may be refined in regions of interest where a greater accuracy in the description of the geometry may be required. While the anatomical model may be represented initially in pixel units, a conversion into physical units is required. This is performed by taking into account the pixel spacing in physical units, and the magnification factor. Then, the FFR value can be calculated. This can be based on computational fluid dynamics (CFD) analytical model or a machine-learning model, to give two examples.

The CFD model may be a 1-D - 0-D reduced-order geometrical multiscale model. A time-varying flow rate profile is applied as inlet boundary condition, and three-element windkessel models are used as outlet boundary conditions. Left and right coronary artery (LCA, RCA) flow rate profiles differ due to the different influence of the myocardial contraction on the circulation: systolic flow is markedly lower in the LCA than in the RCA, when compared to the diastolic flow. Hence, two separate typical, normalized time-varying flow rate profiles for LCA and RCA may be used. For the patient-specific computations these normalized profiles are scaled by an average flow rate value. Stenosis detection is automatically performed through an iterative algorithm which is initialized based on the user-defined markers. A semi-analytical pressure drop model, parameterized by the geometry, may be employed to compute blood flow for these segments.

Next, the machine-learning model is discussed. This can include the following steps: first, generation of synthetic coronary arterial trees; second, CFD based approach for computing FFR in coronary arteries; third, feature definition and extraction for generating the mapping between the coronary anatomy and FFR. While the training process is based solely on synthetic arterial trees, FFR is computed during the online prediction phase for patient-specific coronary anatomies. This step is fully automated, consisting of feature extraction, and application of the pre-learned model to compute FFR at all locations of the coronary tree. The synthetic coronary arterial trees used for setting up the training database are generated algorithmically in three stages. During the first stage, the structure of the coronary tree is determined, i.e. number of generations and number of segments. During the second stage, first the length of each segment is set, and, next, the vessel radius at each location is defined (including tapering). These properties are determined by a set of parameters, whose values are randomly sampled in a pre-defined interval. The first two stages enable the generation of healthy coronary anatomical models. The third stage inserts stenoses into the coronary trees. Each stenosis is defined by a set of parameters: percentage diameter stenosis, stenosis length, stenosis center, minimum radius stenosis region length, proximal - distal radius variation (radius tapering). Both single branch and bifurcation stenoses are generated. The Medina classification is employed In case of bifurcation stenoses, and for each stenosed bifurcation segment the above mentioned parameters are independently set. The methodology described above covers the generation of the input data in the training database. To generate the corresponding output, a reduced-order multiscale fluid-structure interaction hemodynamic model is employed to compute the CFD based FFR values, as discussed above. Then, an ML algorithm capable of predicting FFR independently at any centerline location in the anatomical model. Thus, a set of features is defined independently at each centerline location. Since local coronary hemodynamics are influenced by both the local and the proximal and distal anatomy, we define features based on local, proximal and distal anatomical characteristics. The coronary circulation has a tree like structure, and, thus, there is a single upstream path, but typically multiple downstream paths. Hence, to define the features of the distal anatomy, we choose the so called main downstream path, determined based on the healthy reference radius, the number and the length of downstream branches. A deep neural network containing multiple hidden layers is used as machine learning model. Each neuron in each layer is connected to all neurons in the next layer, i.e. a fully connected architecture is employed. Several features are extracted from the anatomical model for each location, and connected to the input layer of the network. Next, examples with respect to the input to the deep neural network are discussed (i.e., intermediate processing results of the processing pipelines). Some features that are input to the deep neural network characterize the anatomy only at the specific location at which the FFR prediction is performed. Examples are actual radius of the vessel, and the reference radius of the hypothetically healthy vessel (if the current location is not stenoses, the two values are identical). Additionally, a branch specific ischemic weight can be used, representing the potential contribution of the branch to the overall ischemic state of the individual. Its value is initially set based on the reference radiuses of all branches in the anatomical model, and then adapted as described below.

Beyond such local features, it is also possible to input, to the deep-neural network, features defined based on the proximal and distal vasculature. This is explained next. A first step in the definition of the proximal and distal features is the identification of all proximal and distal stenoses. Stenoses are identified fully automatically: all narrowings with a radius reduction larger than 10% are marked as stenoses. Next, all identified stenoses are ranked based on the degree of radius reduction, and the most severe four proximal and distal stenoses are retained. For each stenosis it is possible to compute the following anatomical characteristics and their non-linear product combinations as intermediate processing results:

Minimum radius

Proximal radius

Distal radius

Length of the stenotic segment with minimum radius

Total stenotic length

$$\%DR = \left(1 - \frac{r_{sten}}{\left(r_{prox} + r_{dist}\right)/2}\right) \cdot 100$$

Radius reduction [%]:

where $r_{prox}$ and $r_{dist}$ are the proximal and respectively distal radiuses of the stenosis, while $r_{prox}$ is the smallest stenotic radius

Since the threshold is set at 10%, the calculation takes into consideration also very mild stenoses, which, taken separately, have a small ischemic effect, but, when cumulated, may lead to a functionally significant ischemic state. Additionally, we also define cumulative proximal and distal features, based on the aggregation of the features describe above. The threshold can be tuned.

These are only some examples of intermediate processing results that can be used as input to the machine-learning algorithm. Further examples are listed in TAB. 3 below.

TAB. 3: Intermediate processing results of the processing pipelines computed at each point along the coronary 2-D centerline, which are used as inputs to the machine-learning based FFR algorithm to compute $FFR_{ML}$ at that point. Each feature has been classified as quantifying upstream, local or downstream information.

|   | Intermediate processing result | Type |
|---|---|---|
| 1 | Distal radius of most significant stenosis upstream | Upstream |
| 2 | Minimum radius of most significant stenosis upstream | Upstream |
| 3 | Reference radius of branch containing most significant stenosis upstream | Upstream |
| 4 | Percentage diameter stenosis of most significant stenosis upstream | Upstream |
| 5 | Total length of most significant stenosis upstream | Upstream |
| 6 | Minimum radius length of most significant stenosis upstream | Upstream |
| 7 | Entrance length of most significant stenosis upstream | Upstream |

(continued)

|  | Intermediate processing result | Type |
|---|---|---|
| 8 | Exit length of most significant stenosis upstream | Upstream |
| 9 | Intrinsic ischemic contribution score of most significant stenosis upstream | Upstream |
| 10 | Expansion ischemic contribution score of most significant stenosis upstream | Upstream |
| 11 | Tapering ischemic contribution score of most significant stenosis upstream | Upstream |
| 12 | Total ischemic contribution score of most significant stenosis upstream | Upstream |
| 13 | Percentage diameter stenosis of second most significant stenosis upstream | Upstream |
| 14 | Total ischemic contribution score of second most significant stenosis upstream | Upstream |
| 15 | Percentage diameter stenosis of third most significant stenosis upstream | Upstream |
| 16 | Total ischemic contribution score of third most significant stenosis upstream | Upstream |
| 17 | Percentage diameter stenosis of fourth most significant stenosis upstream | Upstream |
| 18 | Total ischemic contribution score of fourth most significant stenosis upstream | Upstream |
| 19 | Percentage diameter stenosis of most significant stenosis downstream | Downstream |
| 20 | Total ischemic contribution score of most significant stenosis downstream | Downstream |
| 21 | Percentage diameter stenosis of second most significant stenosis downstream | Downstream |
| 22 | Total ischemic contribution score of second most significant stenosis downstream | Downstream |
| 23 | Aggregated ischemic contribution score between ostium and current location | Upstream - Local |
| 24 | Aggregated healthy ischemic contribution score between ostium and current location | Upstream - Local |
| 25 | Aggregated ischemic contribution score between current location and largest downstream outlet | Local - downstream |
| 26 | Aggregated healthy ischemic contribution score between current location and largest downstream outlet | Local - downstream |
| 27 | Ischemic weight of the current coronary segment | Local |

A key concept is the idea of ischemic contribution score, which quantifies the geometric significance of a particular segment to potentially cause ischemia. There are three components of the ischemic contribution score: (i) intrinsic ischemic contribution score $S_i$, (ii) expansion ischemic contribution score $S_e$, which is an additional factor quantifying the effect of sudden vessel enlargements (e.g. latter part of a stenosis), and (iii) tapering ischemic contribution

score $S_t$, which is important for longer lesions and quantifies the role played due to the gradual reduction of the vessel radius. The total ischemic contribution score is the sum of these three components:

$$S_{total} = S_i + S_e + S_t$$

The intrinsic ischemic contribution score $S_i$ quantifies the effect of local variation in geometry to the viscous energy losses experienced by a fluid in a vessel with the given local radius. It is simply defined as the integral of $r^4$ over the region of interest, where r is the local radius of the vessel

$$S_i = w \int_{x_1}^{x_2} r(x)^{-4} dx$$

Here, $x_1$ and $x_2$ are the locations along the centerline which bound the region of interest. The pre-factor term w is present in the definition of all components of the ischemic contribution score, and is the ischemic weight of the segment which will be defined shortly. The intrinsic ischemic contribution score is similar to other features such as lumen length/(lumen diameter)$^4$ which have been used in literature previously [1].

The expansion ischemic contribution score $S_e$ is defined as

$$S_e = w \left( \frac{1}{r_{min}^2} - \frac{1}{r_h^2} \right)^2$$

where, $r_{min}$ is the minimum radius of the stenosis and $r_h$ is the estimated healthy radius of the segment in the absence of stenosis. This feature is inspired by the energy losses experienced by a fluid in a vessel segment with abrupt increase in radius [2].

The tapering ischemic contribution score $S_t$ is defined as

$$S_t = w \left( \frac{1}{r_{distal}^4} - \frac{1}{r_{proximal}^4} \right)$$

where, $r_{distal}$ and $r_{proximal}$ are the distal and proximal radii of the segment of interest.

The concept of ischemic weight is present in all of the definitions given above. This factor looks at what fraction of the entire tree is downstream to the current location. Let $O = \{o_1, o_2, .. o_n\}$ be the set of all outflow locations in the coronary tree. We define $O(x)$ to be the set of all outflow locations, which are distal to the current location $x$. Naturally, $O(x)$ is a subset of the total set $O$. The ischemic weight is defined as

$$w = w_{total} \frac{\sum_{o \in O(x)} r^3}{\sum_{o \in O} r^3}$$

where $w_{total}$ represents the ischemic weight at the root of the arterial tree and is estimated based on the healthy radius of the root segment. The ischemic weight has the geometric interpretation of being related to the fraction of myocardium which is being perfused by blood passing through the current location in the coronary tree.

It is worth noting that all of the features above are purely geometric, and are related to the radii of the different vessels. Together, these features present a complete picture of the hemodynamic effect of the local, upstream and downstream properties of the coronary tree at the current location, permitting an accurate computation of the fractional flow reserve at each point of the coronary tree.

All the above-described intermediate processing results that serve as input the deep neural network (as an example implementation of a machine-learning analysis algorithm) rely on the information extracted from the 2-D image and are based on the radiuses, and the centerline length. To further improve the prediction performance, the centerline length may be corrected, to account for the foreshortening present in the 2-D image.

Additional ML based models may be employed to generate other relevant input features for the ML based FFR prediction: Stenosis pressure drop (separate models may be employed for different types of stenoses: focal, diffuse, tandem, bifurcation, etc.); or curvature effect (assessing the severity of tortuosity).

Above, two options - CFD analytical model-based and machine-learning model based - for calculating the first-order estimates 231-234 of the FFR have been disclosed. The implementation of the respective processing pipelines 201-204 has been disclosed and the respective intermediate processing results 221-224 have been disclosed. Then, it is possible to consolidate the multiple first-order estimates 231-234 of the FFR to determine the second-order estimate 240 of the FFR.

In the illustrated example of FIG. 9, a multi-stage consolidation is employed. In a first stage 281, one or more hard filters are applied. This means that the contribution of certain first-order estimates 231-234 to the second-order estimate 240 is fully suppressed. For example, the illustrated example of FIG. 9, the contribution of the first-order estimate 232 is suppressed. This could be based on, e.g., one or more comparative metrics associated with the first-order estimates 231-234 and/or one or more of the intermediate processing results 221-224. For instance, a statistical distribution of the first-order estimates 231-234 may be determined and an outlier detection could be used to identify the first-order estimate 232 as an outlier. Alternatively or additionally, it would be possible to calculate one or more similarity measures between intermediate processing results 221-224 and identify the first-order estimate 232 as outlier; respective aspects have been discussed above, in connection with FIG. 8 in FIG. 9. Respective intermediate processing results that can serve as a basis for hard filtering have been listed above in connection with TAB. 3 and TAB. 2. According to examples, where the 2-D images 211-214 are associated with multiple views, it would be possible to determine any position-based similarity measure in accordance with deformation fields between those views. Alternatively or additionally to such comparative metrics that are operating based on the intermediate processing results and/or the first-order estimates, it would be, alternatively or additionally, also be possible that the consolidating is based on a user input and/or an image quality score associated with each one of the input images 211-214 and/or an uncertainty level associated with the first-order estimates 231-234.

FIG. 9 also illustrates a second stage 282 of the consolidation of the first-order estimates 231-234 of the FFR. This second stage 282 corresponds to a soft weighting of the first-order estimates 231-234 that remain after the first stage 281. In other words, it would be possible to determine weights for each one of the first-order estimates 231-234. Again, these weights could be determined based on one or more of the following: an image quality score associated with each one of the multiple 2-D images 211-214; one or more comparative metrics that are based on similarity measures between one or more intermediate processing results 221-224, a distance to a most probable value of a statistical distribution, etc.. For instance, these weights could then be used at 283 to calculate a weighted combination, e.g., weighted average. Instead of such a weighted aggregation, it would also be possible to determine a weighted distribution using a statistical analysis and then used, e.g., a most probable value of the weighted distribution as the second-order estimate 240.

Summarizing, above, techniques have been disclosed which enable to estimate a hemodynamic property such as the FFR value. The estimation is based on multiple processing pipelines associated with multiple 2-D images. Thus, even though an inherently 3D hemodynamic property such as the FFR may be calculated, it is possible to implement processing of individual 2-D images. Thus, single frame metrics can be employed for such analysis. To nonetheless, yield an accurate and robust results for the hemodynamic property such as the FFR value it is then possible to consolidate the multiple first-order estimates obtained from single metric evaluation of the 2-D images. The consolidation can take into account that certain first-order estimates may be inaccurate and/or represent outliers, due to the calculation restricted to 2-D that does not take into account inherently 3-D relationships between multiple views or multiple features.

Further summarizing, at least the following EXAMPLES have been disclosed:

EXAMPLE 1. A computer-implemented method, comprising:

- obtaining multiple two-dimensional images (151, 211, 212, 213, 214) of an angiographic X-ray examination of a coronary system,
- for each one of the multiple two-dimensional images (151, 211, 212, 213, 214): in a respective processing pipeline (201, 202, 203, 204), applying one or more analysis algorithms (291, 292, 293) to the respective one of the multiple two-dimensional images (151, 211, 212, 213, 214) to obtain a respective first-order estimate (231, 232, 233, 234) of a hemodynamic property of the coronary system, and
- determining at least one second-order estimate (240) of the hemodynamic property by consolidating the first-order estimates (231, 232, 233, 234) of the hemodynamic property obtained for each one of the multiple two-dimensional images (151, 211, 212, 213, 214).

EXAMPLE 2. The computer-implemented method of EXAMPLE 1,

wherein an intermediate processing result of the one or more analysis algorithms (291, 292, 293) comprises a length of a vessel of the coronary system,

wherein the length of the vessel is compensated with respect to a perspective foreshortening associated with a respective view of the respective two-dimensional image.

EXAMPLE 3. The computer-implemented method of EXAMPLE 1 or 2,
wherein said consolidating is based on one or more comparative metrics that compare one or more data structures of the processing pipelines (201, 202, 203, 204) associated the multiple two-dimensional images (151, 211, 212, 213, 214) .

EXAMPLE 4. The computer-implemented method of EXAMPLE 3,
wherein the one or more data structures comprise the multiple two-dimensional images (151, 211, 212, 213, 214).

EXAMPLE 5. The computer-implemented method of EXAMPLE 3 or 4,
wherein the one or more data structures comprise at least one intermediate processing result of the one or more analysis algorithms (291, 292, 293).

EXAMPLE 6. The computer-implemented method of any one of EXAMPLEs 3 to 5,
wherein the one or more data structures comprise the first-order estimates (231, 232, 233, 234) of the hemodynamic property.

EXAMPLE 7. The computer-implemented method of any one of EXAMPLEs 3 to 6,

wherein the one or more comparative metrics comprise a statistical analysis of a distribution of the of the first-order estimates (231, 232, 233, 234) of the hemodynamic property,

wherein the at least one second-order estimate (240) of the hemodynamic property is determined based on a most probable value of the distribution.

EXAMPLE 8. The computer-implemented method of EXAMPLE 7,
wherein the distribution is weighted based on a further one of the one or more comparative metrics.

EXAMPLE 9. The computer-implemented method of any one of EXAMPLEs 3 to 8,
wherein the one or more comparative metrics comprise a statistical analysis of a distribution of the first-order estimates (231, 232, 233, 234) of the hemodynamic property.

EXAMPLE 10. The computer-implemented method of EXAMPLE 9,
wherein the one or more comparative metrics comprise an outlier detection that is based on the statistical analysis of the distribution.

EXAMPLE 11. The computer-implemented method of any one of EXAMPLEs 3 to 10,
wherein the one or more comparative metrics comprise at least one similarity measure between at least one of pairs of the first-order estimates (231, 232, 233, 234) of the hemodynamic properties or pairs of intermediate processing results of the one or more analysis algorithms (291, 292, 293) .

EXAMPLE 12. The computer-implemented method of EXAMPLE 11,
wherein the intermediate processing results are selected from a group comprising: a length of a main branch of vessels the coronary system; an average diameter of a selected one of a vessel of the coronary system; a maximum diameter of the main branch of the coronary system; a maximum diameter of a stenosis at the main branch of the coronary system; an integral computed along the main branch of the coronary system; a number of detected stenosis; a position of a proximal clipping point; a position of a distal clipping point; a position of a catheter tip; a position of centerline trace of the main branch of the coronary system; a number of bifurcations of the coronary system; positions of the bifurcations; a number of branches of the coronary system; a position of the branches of the coronary system; a number of detected stenoses; a position of the detected stenosis; a consistency between heat maps.

EXAMPLE 13. The computer-implemented method of EXAMPLE 11 or 12,

wherein the at least one similarity measure comprises a position-based similarity measure,
wherein the position-based similarity measure is determined in accordance with deformation fields between at least one of views or timestamps associated with respective pairs of the images (151, 211, 212, 213, 214).

EXAMPLE 14. The computer-implemented method of any one of the preceding EXAMPLEs,
wherein said consolidating is based on a weighted combination of the first-order estimates (231, 232, 233, 234) of the hemodynamic property.

EXAMPLE 15. The computer-implemented method of EXAMPLE 14, and of any one of EXAMPLEs 3 to 13,
wherein weights of the weighted combination are set based on the one or more comparative metrics.

EXAMPLE 16. The computer-implemented method of EXAMPLE 15,
wherein at least one of the one or more comparative metrics sets the respective weight to suppress any impact of the respective first-order estimate (231, 232, 233, 234) of the hemodynamic property on the at least one second-order estimate (240) of the hemodynamic property.

EXAMPLE 17. The computer-implemented method of EXAMPLE 15 or 16,
wherein at least one of the one or more comparative metrics sets the respective weight to increase or decrease the impact of the respective first-order estimate (231, 232, 233, 234) of the hemodynamic property on the at least one second-order estimate (240) of the hemodynamic property.

EXAMPLE 18. The computer-implemented method of any one of the preceding EXAMPLEs,
wherein said consolidating is based on an image quality score determined for each one of the multiple images (151, 211, 212, 213, 214).

EXAMPLE 19. The computer-implemented method of any one of the preceding EXAMPLEs,
wherein said consolidating is based on at least one of an uncertainty level associated with the first-order estimates (231, 232, 233, 234) of the hemodynamic property, or an out-of-distribution likelihood score of inputs to the one or more analysis algorithms.

EXAMPLE 20. The computer-implemented method of any one of the preceding EXAMPLEs,
wherein said consolidating is based on a user input obtained from a human-machine interface.

21. The computer-implemented method of any one of the preceding EXAMPLEs,
wherein the one or more analysis algorithms (291, 292, 293) are single frame metrics that operate based on individual ones of the multiple two-dimensional images (151, 211, 212, 213, 214).

EXAMPLE 22. The computer-implemented method of any one of the preceding EXAMPLEs,
wherein the multiple two-dimensional images (151, 211, 212, 213, 214) are associated with multiple views of the coronary system.

EXAMPLE 23. The computer-implemented method of any one of the preceding EXAMPLEs,
wherein the multiple two-dimensional images (151, 211, 212, 213, 214) are associated with multiple time-points with respect to a contrast medium bolus.

EXAMPLE 24. The computer-implemented method of any one of the preceding EXAMPLEs,

wherein multiple second-order estimates (240) of the hemodynamic property are determined,
wherein the method further comprises:

- ranking the multiple second-order estimates (240), and
- configuring a user interface to present the multiple second-order estimates (240) in accordance with said ranking.

EXAMPLE 25. The computer-implemented method of EXAMPLE 24, and of any one of EXAMPLEs 3 to 17,
wherein said ranking is based on the one or more comparative metrics.

EXAMPLE 26. The computer-implemented method of any one of the preceding EXAMPLEs,

wherein multiple second-order estimates (240) are determined,

wherein the method further comprises:

- determining one or more clusters in a distribution of the multiple second-order estimates (240), and
- configuring a user interface presenting the multiple second-order estimates (240) in accordance with the one or more clusters.

EXAMPLE 27. The computer-implemented method of any one of the preceding EXAMPLEs,
wherein the hemodynamic property comprises a fractional flow reserve value for a stenosis of the coronary system.

EXAMPLE 28. The computer-implemented method of any one of the preceding EXAMPLEs,
wherein the hemodynamic property comprises at least one of branch labels for coronary arteries of the coronary system, a segmentation between vessel and background, a topology of the coronary arteries of the coronary system, or a stenosis detection.

EXAMPLE 29. The method of any one of the preceding EXAMPLES,
wherein the at least one second-order estimate (240) is determined further based on another estimate of the hemodynamic property obtained from another angiographic examination.

EXAMPLE 30. The method of any one of the preceding EXAMPLEs, further comprising:

- re-parameterizing the one or more analysis algorithms (291, 292, 293) based on the at least one second-order estimate (240) of the hemodynamic property.

EXAMPLE 31. A processing device comprising at least one processor and a memory, the at least one processor being configured to load program code from the memory and to execute the program code, wherein execution of the program code causes the at least one processor to perform the method of any one of the preceding EXAMPLEs.

EXAMPLE 32. A computer program comprising program code, execution of the program code causing at least one processor to perform the method of any one of EXAMPLEs 1 to 30.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

**Claims**

1. A computer-implemented method, comprising:

    - obtaining multiple two-dimensional images (151, 211, 212, 213, 214) of an angiographic X-ray examination of a coronary system,
    - for each one of the multiple two-dimensional images (151, 211, 212, 213, 214): in a respective processing pipeline (201, 202, 203, 204), applying one or more analysis algorithms (291, 292, 293) to the respective one of the multiple two-dimensional images (151, 211, 212, 213, 214) to obtain a respective first-order estimate (231, 232, 233, 234) of a hemodynamic property of the coronary system, and
    - determining at least one second-order estimate (240) of the hemodynamic property by consolidating the first-order estimates (231, 232, 233, 234) of the hemodynamic property obtained for each one of the multiple two-dimensional images (151, 211, 212, 213, 214).

2. The computer-implemented method of claim 1,
wherein an intermediate processing result of the one or more analysis algorithms (291, 292, 293) comprises a length of a vessel of the coronary system,
wherein the length of the vessel is compensated with respect to a perspective foreshortening associated with a respective view of the respective two-dimensional image.

3. The computer-implemented method of claim 1 or 2,
wherein said consolidating is based on one or more comparative metrics that compare one or more data structures of the processing pipelines (201, 202, 203, 204) associated the multiple two-dimensional images (151, 211, 212, 213, 214) .

4. The computer-implemented method of claim 3,
    wherein the one or more data structures comprise the multiple two-dimensional images (151, 211, 212, 213, 214).

5. The computer-implemented method of claim 3 or 4,
    wherein the one or more data structures comprise at least one intermediate processing result of the one or more analysis algorithms (291, 292, 293).

6. The computer-implemented method of any one of claims 3 to 5,
    wherein the one or more data structures comprise the first-order estimates (231, 232, 233, 234) of the hemodynamic property.

7. The computer-implemented method of any one of claims 3 to 6,

    wherein the one or more comparative metrics comprise a statistical analysis of a distribution of the of the first-order estimates (231, 232, 233, 234) of the hemodynamic property,
    wherein the at least one second-order estimate (240) of the hemodynamic property is determined based on a most probable value of the distribution.

8. The computer-implemented method of any one of claims 3 to 7,
    wherein the one or more comparative metrics comprise a statistical analysis of a distribution of the first-order estimates (231, 232, 233, 234) of the hemodynamic property.

9. The computer-implemented method of any one of claims 3 to 8,
    wherein the one or more comparative metrics comprise at least one similarity measure between at least one of pairs of the first-order estimates (231, 232, 233, 234) of the hemodynamic properties or pairs of intermediate processing results of the one or more analysis algorithms (291, 292, 293).

10. The computer-implemented method of claim 9,

    wherein the at least one similarity measure comprises a position-based similarity measure,
    wherein the position-based similarity measure is determined in accordance with deformation fields between at least one of views or timestamps associated with respective pairs of the images (151, 211, 212, 213, 214).

11. The computer-implemented method of any one of the preceding claims,
    wherein said consolidating is based on a weighted combination of the first-order estimates (231, 232, 233, 234) of the hemodynamic property.

12. The computer-implemented method of any one of the preceding claims,
    wherein said consolidating is based on an image quality score determined for each one of the multiple images (151, 211, 212, 213, 214).

13. The computer-implemented method of any one of the preceding claims,
    wherein said consolidating is based on at least one of an uncertainty level associated with the first-order estimates (231, 232, 233, 234) of the hemodynamic property, or an out-of-distribution likelihood score of inputs to the one or more analysis algorithms.

14. The computer-implemented method of any one of the preceding claims,
    wherein the one or more analysis algorithms (291, 292, 293) are single frame metrics that operate based on individual ones of the multiple two-dimensional images (151, 211, 212, 213, 214).

15. The computer-implemented method of any one of the preceding claims,
    wherein the multiple two-dimensional images (151, 211, 212, 213, 214) are associated with multiple views of the coronary system.

FIG 1

151

151 — 155

151 — 156

FIG 2

FIG 3

FIG 4

FIG 5

FIG 6

FIG 7

FIG 8

FIG 9

| | 361 | 362 | 363 | 364 | 365 |
|---|---|---|---|---|---|
| 361 | | | | | |
| 362 | | | | | |
| 363 | | | | | |
| 364 | | | | | |
| 365 | | | | | |

| | very similar |
|---|---|
| | similar |
| | dissimilar |
| | very dissimilar |

FIG 10

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BOURANTAS CHRISTOS V. ET AL: "Comparison of Quantitative Coronary Angiography with Intracoronary Ultrasound. Can Quantitative Coronary Angiography Accurately Estimate the Severity of a Luminal Stenosis?", ANGIOLOGY, vol. 60, no. 2, 28 May 2008 (2008-05-28), pages 169-179, XP93043451, US ISSN: 0003-3197, DOI: 10.1177/0003319708317338 Retrieved from the Internet: URL:http://journals.sagepub.com/doi/pdf/10.1177/0003319708317338> | 1,14,15 | INV. G06T7/00 |
| A | * abstract * * figure 2 * * pages 169-172 * | 2-13 | |
| X | M'HIRI FATEN ET AL: "Automatic evaluation of vessel diameter variation from 2D X-ray angiography", INTERNATIONAL JOURNAL OF COMPUTER ASSISTED RADIOLOGY AND SURGERY, SPRINGER, DE, vol. 12, no. 11, 13 July 2017 (2017-07-13), pages 1867-1876, XP036348661, ISSN: 1861-6410, DOI: 10.1007/S11548-017-1639-9 [retrieved on 2017-07-13] | 1,14,15 | TECHNICAL FIELDS SEARCHED (IPC) G06T |
| A | * abstract * * figure 1 * * pages 1868-187 * | 2-13 | |

The present search report has been drawn up for all claims

| Place of search | Date of completion of the search | Examiner |
|---|---|---|
| Munich | 2 May 2023 | Eveno, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................
& : member of the same patent family, corresponding document

EPO FORM 1503 03.82 (P04C01)